Europäisches Patentamt

European Patent Office

Office européen des brevets

(19)

(11) Numéro de publication: **0 008 547**
**B1**

(12) **FASCICULE DE BREVET EUROPEEN**

(45) Date de publication du fascicule du brevet: **10.11.82**

(51) Int. Cl.³: **C 07 D 233/78**

(21) Numéro de dépôt: **79400533.0**

(22) Date de dépôt: **26.07.79**

(54) **Procédé de fabrication de la R.S. p-hydroxyphényl-5 imidazolidinedione-2,4 ou R.S. p-hydroxyphényl-5 hydantoine.**

(30) Priorité: **03.08.78 FR 7822989**

(43) Date de publication de la demande:
**05.03.80 Bulletin 80/5**

(45) Mention de la délivrance du brevet:
**10.11.82 Bulletin 82/45**

(84) Etats contractants désignés:
**AT BE CH DE FR GB IT NL SE**

(56) Documents cités:
**BE - A - 865 027**

**JOURNAL OF THE AMERICAN CHEMICAL SOCIETY, Vol. 73, Octobre 1961, WASHINGTON (US)
J. C. SHEEBAN et al.: "The Synthesis of Substituted Penicillins and Simpler Structural Analogs. V. The Application of 5-PHENYLOXAZOLIDINE-2,4-DIONES to the synthesis of Phenylacetylamino-beta-lactams", pages 4752 à 4755.**

(73) Titulaire: **SOCIETE FRANCAISE HOECHST Société anonyme dite:
3, avenue du Général de Gaulle
F-92800 Puteaux (FR)**

(72) Inventeur: **Christidis. Yani
12 rue de Constantinople
F-75008 Paris (FR)**

(74) Mandataire: **Rinuy, Guy et al,
Cabinet Rinuy et Santarelli 14, Avenue de la Grande Armée
F-75017 Paris (FR)**

Procédé de fabrication de la R.S. p-hydroxyphényl-5 imidazolidinedione-2,4
ou R.S. p-hydroxyphényl-5 hydantoïne

La présente invention concerne un procédé de préparation de la R.S. p-hydroxyphényl-5 imidazolidinedione-2,4 ou R.S. p-hydroxyphényl-5 hydantoïne.

La R.S. p-hydroxyphényl-5 imidazolidinedione-2,4 ou R.S. p-hydroxyphényl-5 hydantoïne est utilisée pour accéder soit à l'acide R.S. amino-2-p-hydroxyphénylacétique soit, après dédoublement, à l'acide R amino-2-p-hydroxy-phénylacétique largement utilisé actuellement dans la préparation des pénicillines semi-synthétiques.

La R.S. p-hydroxyphényl-5 hydantoïne peut être obtenue soit par déméthylation par l'acide bromhydrique à 48% dans l'acide acétique à 100°C de la R.S. p-méthoxyphényl-5 hydantoïne issue de la cyclisation en milieu acide chlorhydrique de l'acide R.S. uréido-2-p-méthoxyphénylacétique préparé par condensation du cyanate de potassium sur le chlorhydrate de l'acide R.S. amino-2-p-méthoxyphénylacétique préparé par condensation du cyanate de potassium sur le chlorhydrate de l'acide R.S. amino-2-p-méthoxyphé-nylacétique (R. M. Coghill et T. B. Johnson, J. Amer. Chem. Soc. *47*, 191 (1925), J. Eagles et collaborateurs, J. Biol. Chem. *121*, 425 (1971), soit en effectuant sur le p-hydroxybenzaldéhyde la réaction de Bucherer (H. R. Henze et R. J. Speer, J. Amer. Chem. Soc. *64*, 522 (1942); E. K. Harvill et R. M. Herbst, J. Org. Chem. *9*, 21 (1944); R. Bognar et collaborateurs, Antibiotiki *9*, 875 (1974) Chem. Abst. *62*, 4105 c (1965), soit enfin par réaction, en milieu acide et à chaud, de l'acide glyoxylique et de l'urée sur le phénol suivie d'un traitement pour séparer la R.S. p-hydroxyphényl-5 hydantoïne formée de son isomère la R.S. p-hydroxyphényl-5 hydantoïne obtenue parallélement en faible quantité (voir le brevet belge N° 865.027).

Plus généralement, on sait préparer les R.S. aryl-5 hydantoïnes à partir des alpha-amino acides par cyclisation des acides R.S. aryl-2-hydantoïques correspondants. Ces acides hydantoïques sont eux-même obtenus soit en faisant réagir un cyanate alcalin sur le sulfate ou le chlorhydrate de l'alpha-amino acide (F. Urech — Annalen *164*, 268 (1872); R. Gaudry Can. J. *29*, 584 (1951)), soit en chauffant l'acide alpha-aminé avec l'urée à une température de 120 à 130°C (W. Heintz, Annalen *133*, 65 (1865)), soit enfin en faisant réagir ces deux substances en présence d'hydroxyde de baryum dans l'eau (W. Wislicenus, Annalen *165*, 103 (1873)).

Il est aussi possible d'obtenir les R.S. aryl-5 hydantoïnes à partir des aldéhydes aromatiques par la réaction de Bucherer-Bergs (H. Bergs brevet allemand N° 566 094 (1929); H. T. Bucherer et V. A. Lieb, J. prakt. Chem. *141*, 5 (1934), mais on peut aussi remplacer le dérivé carbonylé soit par sa combinaison bisulfitique (J. Klosa, Arch. Pharm. *285*, 278 (1952), soit par sa cyanhydrine (H. T. Bucherer et W. Steiner, J. Prakt. Chem. *140*, 291 (1934) soit enfin par un de ses dérivés fonctionnels: oxime, semi-carbazone, thiosemicarbazone, phényl-hydrazone, bases de Schiff, ... (N. N. Coker et collaborateurs, J. Org. Chem. *27*, 3201 (1962)).

Une autre méthode de préparation des R.S. aryl-5 hydantoïnes consiste à condenser des arylglyoxals sur l'urée, mais très souvent ces cyclisations prennent une allure complexe (H. J. Fischer et collaborateurs, J. Amer. Chem. Soc. *64*, 1434 (1942)).

Enfin, il est possible d'accéder aux R.S. aryl-5 hydantoïnes par action, en milieu acide, d'un aryle sur la R.S. alkoxy-5 hydantoïne. Lorsque le dérivé aromatique est monosubstitué, on isole en fin de réaction un mélange d'isomères ortho et para qu'il est nécessaire de purifier (G. Benet et D. Ben Ishai-Chem. Comm. *1969*, 376).

En résumé, les méthodes connues permettent d'obtenir les R.S. aryl-5 hydantoïnes soit à partir d'acides alpha-aminés racémiques, soit à partir d'aldéhydes aromatiques ou de leurs dérivés fonctionnels, soit à partir d'arylglyoxal, soit à partir de R.S. alkoxy-5 hydantoïnes, soit enfin à partir du phénol, d'acide glyoxylique et d'urée.

Or, la majorité de ces matières sont d'un accès difficile, laborieux et onéreux. en particulier, le p-hydroxybenzaldéhyde n'est obtenu qu'après des opérations de purifications longues et compliquées par réaction de Reimer-Tiemann sur le phénol (voir le brevet des Etats-Unis d'Amérique N° 3.365.500 (1968).

Lorsqu'on utilise la méthode directe de synthèse à partir de l'acide glyoxylique, du phénol et de l'urée, il est nécessaire, en raison de la non-régiosélectivité de cette condensation, d'effectuer, en fin de réaction, un traitement de purification pour isoler la R.S. p-hydroxyphényl-5 hydantoïne pure exempte de l'isomère o-hydroxyphényl-5 hydantoïne.

Or, la demanderesse a trouvé de façon surprenante que l'on pouvait obtenir, avec de bons rendements, la R.S. p-hydroxyphényl-5 hydantoïne en condensant à chaud, en milieu acide, l'acide R.S. p-hydroxymandélique sur l'urée en excès. Il est connu que la condensation de l'urée sur l'acide mandélique racémique ou ses esters conduit, en présence de bases fortes, à des R.S. phényl-5 oxazolidines dione-2,4 (J. C. Shehan et collaborateurs-J. Amer. Soc. *73*, 4752 (1951) et références citées dans l'article).

Selon le procédé de l'invention, en chauffant pendant quelques heures, l'acide R.S. p-hydroxymandélique anhydre ou monohydraté avec l'urée en excès, en présence ou non d'un solvant, puis en introduisant dans le milieu réactionnel débarrassé ou non de son solvant, un acide minéral ou organique, en solution aqueuse ou non, et ensuite en pour suivant le chauffage pendant quelques heures, on isole avec de bons rendements la R.S. p-hydroxyphényl-5 hydantoïne.

Pour une mole d'acide R.S. p-hydroxymandélique monohydraté, on utilise 1 à 7 moles d'urée. On emploie comme acides: l'acide chlorhydrique, l'acide sulfurique, l'acide nitrique, l'acide phosphorique, l'acide p-toluènesulfonique monohydraté, l'acide acétique, mais cette liste n'est pas limitative. La solution aqueuse acide est généralement 5N, mais on peut utiliser une normalité plus faible ou même un acide minéral concentré, le pH du milieu réactionnel étant maintenu au moyen de cet acide à une valeur inférieure à 4.

Lorsqu'on travaille en milieu solvant, il est souhaitable, pour améliorer le rendement, de l'éliminer avant d'introduire la solution aqueuse acide. Les solvants couramment utilisés sont l'acide acétique, l'acide formique.

Lorsque la réaction est effectuée au sein de l'acide acétique, il est avantageux mais non indispensable d'introduire une solution aqueuse d'acide fort.

De même, il est avantageux, en fin de réaction, d'éliminer l'acide introduit soit par distillation sous vide lorsque l'acide introduit est volatil, soit par neutralisation avec une solution aqueuse d'un bicarbonate alcalin.

Certains acides donnent un sel insoluble dans le milieu réactionnel; en fin de réaction, ce sel est filtré puis déplacé par un solution aqueuse d'une bicarbonate alcalin.

Ainsi qu'il à déjà été mentionné, par hydrolyse de la R.S. p-hydroxyphényl-5 hydantoïne, on obtient la p-hydroxyphényl-glycine racémique, largement utilisée dans la préparation des pénicillines semi-synthétiques.

Les exemples suivants sont donnés à titre illustratif et nullement limitatif de l'invention.

### Exemple 1

On chauffe 4 heures à 90°C, 18,6 g (0,1 mole) d'acide R.S. p-hydroxymandélique monohydraté avec 30 g (0,5 mole) d'urée puis, après refroidissement, on introduit dans le milieu réactionnel 100 cm³ d'acide chlorhydrique 5N et on poursuit ensuite le chauffage pendant 3 heures au reflux. On isole ainsi, après refroidissement, 15 g (rendement 78,1%) de R.S. p-hydroxyphényl-5 hydantoïne possédant un point de fusion en tube de 263°C.

### Exemple 2

On chauffe 5 heures à 90°C, 18,6 g (0,1 mole) d'acide R.S. p-hydroxymandélique monohydraté avec 30 g (0,5 mole) d'urée puis, après refroidissement, on introduit dans le milieu réactionnel 100 cm³ d'acide chlorhydrique 5N et on poursuit ensuite le chauffage pendant 17 heures au reflux. La solution obtenue est ensuite concentrée à sec sous vide, puis le résidu cristallisé est repris avec 50 cm³ d'eau. On isole ainsi 16 g (rendement 83,3%) de R.S. p-hydroxyphényl-5 hydantoïne possédant un point de fusion en tube de 261—262°C.

### Exemple 3

On chauffe 150 minutes à 90°C, 18,6 g (0,1 mole) d'acide R.S. p-hydroxymandélique monohydraté avec 30 g (0,5 mole) d'urée puis, après refroidissement, on introduit dans le milieu réactionnel 100 cm³ d'acide sulfurique 5N et on poursuit ensuite le chauffage pendant 1 heure au reflux. On isole ainsi, après refroidissement, 13,1 g (rendement 68,2%) de R.S. p-hydroxyphényl-5 hydantoïne.

### Exemple 4

On opère comme dans l'exemple 3, mais on remplace l'acide sulfurique 5N par de l'acide phosphorique 2,5 M. On obtient ainsi 8,2 g (rendement 42,7%) de R.S. p-hydroxyphényl-5 hydantoïne.

### Exemple 5

On opère comme dans l'exemple 3, mais on remplace l'acide sulfurique 5N par de l'acide nitrique 5N. En fin de réaction, la suspension obtenue est filtrée et le produit cristallisé est repris par une solution aqueuse saturée en bicarbonate de potassium. On isole ainsi 13,5 g (rendement 70,3%) de R.S. p-hydroxyphényl-5 hydantoïne.

### Exemple 6

On chauffe 5 heures à 60°C, 18,6 (0,1 mole) d'acide R.S. p-hydroxymandélique monohydraté, 18 g (0,3 mole) d'urée dans 125 cm³ d'acide acétique cristallisable, puis on introduit 100 cm³ d'acide chlorhydrique 3,5 N et on poursuit ensuite le chauffage pendant 1 heure au reflux. On isole ainsi, après refroidissement, 12 g (rendement 62,5%) de R.S. p-hydroxyphényl-5 hydantoïne.

### Exemple 7

On opère comme dans l'exemple 3 mais on remplace l'acide sulfurique 5N par de l'acide chlorhydrique 7,5N. On obtient ainsi 11,5 g (rendement 59,9%) de R.S. p-hydroxyphényl-5 hydantoïne.

### Exemple 8

On chauffe sous agitation pendant 5 heures à 70°C, 18,6 g (0,1 mole) d'acide R.S. p-hydroxymandélique monohydraté, 30 g (0,5 mole) d'urée dans 125 cm³ d'acide acétique cristallisable. Ensuite, on élimine l'acide acétique par distillation sous vide et le résidu est chauffé pendant 1 heure au reflux dans 100 cm³ d'acide chlorhydrique 5N. On abandonne pendant 16 heures à température ambiante et on filtre le produit cristallisé obtenu. On isole ainsi 14,5 g (rendement 75,5%) de R.S. p-hydroxyphényl-5 hydantoïne.

### Exemple 9

On chauffe 4 heures au reflux 37,2 g (0,2 mole) d'acide R.S. p-hydroxymandélique monohydraté et 24 g (0,4 mole) d'urée dans 250 cm³ d'acide acétique cristallisable, puis on introduit 0,5 cm³ acide chlorhydrique concentré d = 1,18 et ensuite on poursuit le reflux pendant 150 minutes environ. On isole, après refroidissement, 22 g (rendement = 57,42%) de R.S. p-hydroxyphényl-5 hydantoïne.

#### Exemple 10

On chauffe 2 heures au reflux 37,2 g (0,2 mole) d'acide R.S. p-hydroxymandélique, 36,0 g (0,6 mole) d'urée dans 250 cm³ d'acide acétique cristallisable. Après refroidissement, on isole 21,4 g (rendement 55,7%) de R.S. p-hydroxyphényl-5 hydantoïne.

#### Exemple 11

On chauffe 2 heures au reflux 18,6 g (0,1 mole) d'acide R.S. p-hydroxymandélique monohydraté, 18,0 g (0,3 mole) d'urée dans 125 cm³ d'acide acétique cristallisable. Durant la première heure de reflux, on introduit lentement 3,06 g (0,3 mole) d'anhydride acétique. Après refroidissement, on isole 10 g (rendement 52%) de R.S. p-hydroxyphényl hydantoïne.

#### Exemple 12

On chauffe 3 heures à 90°C, 18,6 g (0,1 mole) d'acide R.S. p-hydroxymandélique monohydraté et 12 g (0,2 mole) d'urée dans 70 cm³ d'acide chlorhydrique 2,5N, puis on introduit 10 cm³ d'acide chlorhydrique concentré d = 1,18 et l'on poursuit ensuite le chauffage pendant 2 heures à 90°C. Après refroidissement, on isole 3,5 g (rendement 18,2%) de R.S. p-hydroxyphényl-5 hydantoïne.

#### Exemple 13

On opère comme dans l'exemple 3 mais en remplaçant l'acide sulfurique par 100 cm³ d'acide acétique contenant 2 g d'acide p-toluènesulfonique monohydraté. On obtient ainsi, après refroidissement, 7,6 g (rendement 39,6%) du R.S. p-hydroxyphényl-5 hydantoïne.

Il va de soi que la présente invention n'a été décrite qu'à titre purement explicatif et nullement limitatif et que toute modification utile pourra y être apportée sans sortir de son cadre tel que défini par les revendications ci-après.

#### Revendications

1. Procédé de fabrication de la R.S. p-hydroxyphényl-5 hydantoïne, caractérisé par le fait que l'on condense à chaud, en milieu acide, l'acide R.S. p-hydroxymandélique sur un excès d'urée.

2. Procédé selon la revendication 1, caractérisé par le fait qu'on chauffe l'acide R.S. p-hydroxymandélique anhydre ou monohydraté avec l'urée en excès, en présence ou non d'un solvant, puis on introduit dans le milieu réactionnel débarrassé ou non de son solvant, un acide minéral ou organique en solution aqueuse ou non et ensuite on poursuit le chauffage et on isole la R.S. p-hydroxyphényl-5 hydantoïne résultante.

3. Procédé selon la revendication 2, caractérisé par le fait que l'acide utilisé est choisi parmi les acides chlorhydrique, sulfurique, nitrique, phosphorique, acétique, mélange acide acétique et acide p-toluènesulfonique monohydraté.

4. Procédé selon la revendication 2, caractérisé par le fait que le solvant est choisi parmi les acides acétique et formique.

5. Procédé selon l'une quelconque des revendications 2 à 4, caractérisé par le fait que l'on élimine par distillation le solvant utilisé avant l'introduction de l'acide et que l'on poursuit ensuite le chauffage à une température inférieure ou égale à la température de reflux.

6. Procédé selon l'une quelconque de revendications 1 à 5, caractérisé par le fait que l'acide est une solution aqueuse d'un acide fort.

7. Procédé selon l'une quelconque des revendications 2 à 4, caractérisé par le fait que l'on chauffe à l'état fondu l'acide R.S. p-hydroxymandélique avec un excès d'urée puis qu'on poursuit le chauffage à une température inférieure ou égale à la température de reflux en présence de la solution d'acide fort.

8. Procédé selon l'une quelconque des revendications 2 à 7, caractérisé par le fait qu'en fin de réaction l'acide est éliminé soit par distillation, soit par neutralisation au moyen d'une solution aqueuse de bicarbonate alcalin.

9. Procédé selon l'une quelconque des revendications 6 à 8, caractérisé par le fait que la solution d'acide fort est une solution environ 4 à 5N.

#### Patentansprüche

1. Verfahren zur Herstellung von (R, S)-p-Hydroxyphenyl-5-hydantoin, dadurch gekennzeichnet, dass man in der Wärme im sauren Milieu (R, S)-p-Hydroxymandelsäure mit einem Überschuss Harnstoff kondensiert.

2. Verfahren nach Anspruch 1, dadurch gekennzeichnet, dass man wasserfreie (R, S)-p-Hydroxymandelsäure oder deren Monohydrat mit einem Überschuss Harnstoff in Gegenwart eines Lösungsmittels oder Ohne Lösungsmittel erhitzt, man dann in das vom Lösungsmittel befreite oder nicht befreite Reaktionsmedium eine Mineralsäure oder eine organische Säure, gegebenenfalls in wässriger Lösung, einführt und dann das Erwärmen fortsetzt und das erhaltene (R, S)-p-Hydroxyphenyl-5-hydantoin isoliert.

3. Verfahren nach Anspruch 2, dadurch gekennzeichnet, dass die verwendete Säure aus der Gruppe der Salzsäure, Schwefelsäure, Salpetersäure, Phosphorsäure, Essigsäure und einer Mischung von Essigsäure und p-Toluolsulfosäure-Monohydrat ausgewählt wird.

4. Verfahren nach Anspruch 2, dadurch gekennzeichnet, dass das Lösungsmittel aus der Gruppe Essigsäure und Ameisensäure ausgewählt wird.

5. Verfahren nach einem der Ansprüche 2 bis 4, dadurch gekennzeichnet, dass man das verwendete Lösungsmittel vor der Einführung der Säure durch Destillation entfernt, und man dann die Erwärmung auf einer niedrigeren Temperatur oder der Rückflusstemperatur fortsetzt.

6. Verfahren nach einem der Ansprüche 1 bis 5, dadurch gekennzeichnet, dass die Säure eine wässrige Lösung einer starken Säure ist.

7. Verfahren nach einem der Ansprüche 2 bis 4, dadurch gekennzeichnet, dass man im geschmolzenen Zustand die (R, S)-p-Hydroxy-mandelsäure mit einem Überschuss an Harnstoff erhitzt und dann die Erwärmung bei einer niedrigeren Temperatur oder der Rückflusstemperatur in Gegenwart einer Lösung einer starken Säure fortsetzt.

8. Verfahren nach einem der Ansprüche 1 bis 7, dadurch gekennzeichnet, dass man am Ende der Reaktion die Säure entweder durch Destillation oder durch Neutralisation mit einer wäss-rigen Lösung von alkalischem Hydrogen-carbonat entfernt.

9. Verfahren nach einem der Ansprüche 6 bis 8, dadurch gekennzeichnet, dass die Lösung der starken Säure eine etwa 4 bis 5 N Lösung ist.

## Claims

1. A process of preparation of R.S. 5-p-hydroxyphenyl hydantoin, characterized by hot condensing in an acid medium R.S. p-hydroxymandelic acid on urea in excess.

2. A process according to claim 1, characterized by heating anhydrous or monohydrated R.S. p-hydroxymandelic acid with urea in excess in the presence of a solvent or not, then introducing into the reactional medium freed of its solvent or not, a mineral or organic acid in aqueous or non-aqueous solution and then, continuing the heating and isolating the resulting R.S. 5-p-hydroxyphenyl hydantoin.

3. A process according to claim 2, characterized by selecting the acid used among hydrochloric, sulfuric, nitric, phosphoric, acetic acids, a mixture of acetic acid and monohydrated p-toluenesulfonic acid.

4. A process according to claim 2, characterized by selecting the solvent among the acetic and formic acids.

5. A process according to any of claims 2 to 4, characterized by eliminating by distillation the solvent used before introduction of the acid, and then continuing the heating at a temperature lower than, or equal to, the reflux temperature.

6. A process according to any of claims 1 to 5, characterized in that the acid is an aqueous solution of a strong acid.

7. A process according to any of claims 2 to 4, characterized by heating R.S. p-hydroxymandelic acid in the molten state with urea in excess, then continuing the heating at a temperature lower than, or equal to, the reflux temperature, in the presence of the strong acid solution.

8. A process according to any of claims 2 to 7, characterized by eliminating the acid at the end of the reaction either by distillation or by neutralization by means of an aqueous solution of alkaline bicarbonate.

9. A process according to any of claims 6 to 8, characterized in that the strong acid solution is an about 4 to 5N solution.